# EUROPEAN PATENT APPLICATION

(11) **EP 4 831 194 A1**
(43) Date of publication of application: **28.10.2026**
(21) Application number: 24908357.7
(22) Date of filing: 18.12.2024
(51) Int. Cl.: C07K 14/58, C07K 7/06, A61P 19/00, A23L 33/18

(54) **NATRIURETIC PEPTIDE ANALOG AND USES THEREOF**

(30) Priority: 21.12.2023 KR 20230189038
(71) Applicant: Peptron, Inc., Daejeon 34054 (KR)
(72) Inventor: CHOI, Ho Il, Daejeon 34054 (KR)
(74) Representative: dompatent
(86) International application number: PCT/KR2024/097131
(87) International publication number: WO 2025/136053

(57) **Abstract**

The present disclosure relates to natriuretic peptide analogues with enhanced stability and a pharmaceutical composition comprising the same. A C-type natriuretic peptide analogue according to an embodiment of the present disclosure exhibits a prolonged in vivo half-life, high bioavailability, and a high bone growth rate, and also has a relatively short sequence length, thereby providing advantages in that sequence synthesis is easy and production costs can be reduced. Accordingly, the analogue is expected to be effectively used in compositions for promoting growth and in the prevention or treatment of bone-related diseases.

## Description

### [Technical Field]

The present disclosure relates to natriuretic peptide analogues and uses thereof, and more particularly, to natriuretic peptide analogues with enhanced stability and a pharmaceutical composition comprising the same.

### [Background Art]

Natriuretic peptides found in various animals, including mammals, are generally divided into three types of an atrial natriuretic peptide (ANP), a brain natriuretic peptide (BNP), and a C-type natriuretic peptide (CNP).

The CNP acts as an autocrine and paracrine regulatory mediator released by endothelial cells, cardiomyocytes, and fibroblasts, and performs its role through two cognate receptors, a natriuretic peptide receptor B (NPR-B) and a natriuretic peptide receptor C (NPR-C), which activate respective signaling pathways that mediate separately complementary cellular responses. In addition, the CNP specifically binds to NPR-B to activate guanylyl cyclase-B and regulates an intracellular cGMP level, thereby exerting various physiological activities, such as natriuretic function and homeostasis, and effects of blood pressure regulation, etc.

However, the CNP is rapidly degraded due to its very short half-life of 2.6 minutes in plasma, which is mainly because the CNP is cleaved by neutral endopeptidase (NEP) or degraded due to internalization by a natriuretic peptide receptor C (NPR-C). Therefore, for pharmaceutical use, the development of CNP analogues with a longer half-life than wild-type CNP is required.

Currently, C-type natriuretic peptide analogues developed for commercialization include BMN111 (BioMarin Pharmaceutical Inc.), ASB20123 (Daiichi Sankyo Company, Limited), etc., which have half-lives of approximately 20 minutes and 30 minutes, respectively. Considering that the BMN111 is marketed as a once-daily formulation, there is still a need for the development of CNP analogues that improve characteristics such as half-life, bioavailability, and efficacy, thereby reducing the frequency of administration or improving the convenience of an administration route.

### [Disclosure]

### [Technical Problem]

Accordingly, the present inventors developed a novel C-type natriuretic peptide analogue (CNP analogue) with increased half-life and bioavailability due to enhanced *in vivo* stability and then completed the present disclosure.

Therefore, an object of the present disclosure is to provide a novel C-type natriuretic peptide analogue with increased half-life and bioavailability.

Another object of the present disclosure is to provide a composition for use in promoting growth comprising the C-type natriuretic peptide analogue.

Yet another object of the present disclosure is to provide a pharmaceutical composition for use in preventing or treating growth disorders comprising the C-type natriuretic peptide analogue.

Still another object of the present disclosure is to provide a method for promoting growth using the C-type natriuretic peptide analogue.

Still another object of the present disclosure is to provide a use of the C-type natriuretic peptide analogue for use in promoting growth.

### [Technical Solution]

An aspect of the present disclosure provides a C-type natriuretic peptide analogue having a structure of the following Formula 1:

[Formula 1] (P1)-PPKKGPPNG-(P2)

In Formula 1,
P1 is a peptide consisting of 0 to 4 amino acids, and
P2 is a peptide consisting of the amino acid sequence of SEQ ID NO: 2 or 3, or a peptide comprising one or more amino acid substitutions in the amino acid sequence of SEQ ID NO: 2 or 3.

Another aspect of the present disclosure provides a composition for promoting growth comprising the C-type natriuretic peptide analogue.

Yet another aspect of the present disclosure provides a pharmaceutical composition for use in preventing or treating growth disorders comprising the C-type natriuretic peptide analogue.

Still another aspect of the present disclosure provides a method for promoting growth of a subject other than a human, comprising administering the C-type natriuretic peptide analogue to the subject other than the human.

Still another aspect of the present disclosure provides a use of the C-type natriuretic peptide analogue for promoting growth.

### [Advantageous Effects]

According to an embodiment of the present disclosure, C-type natriuretic peptide analogues have enhanced stability, thereby exhibiting a prolonged in vivo half-life, high bioavailability, and excellent bone growth-promoting effects, and thus can be used for growth promotion or the prevention or treatment of growth disorders.

### [Description of drawings]

FIG. 1 is a graph showing residual amounts measured after administering C-type natriuretic peptide analogues according to an embodiment of the present disclosure to rats.
FIG. 2 is a graph showing half-lives calculated by measuring residual amounts after administering C-type natriuretic peptide analogues according to an embodiment of the present disclosure to rats.
FIG. 3 is a graph showing results of pharmacokinetic evaluation after intravenous administration of C-type natriuretic peptide analogues according to an embodiment of the present disclosure to rats.
FIG. 4 is a graph showing results of pharmacokinetic evaluation after subcutaneous administration of C-type natriuretic peptide analogues according to an embodiment of the present disclosure to rats.
FIG. 5 is a graph showing bioavailability (BA) of C-type natriuretic peptide analogues according to an embodiment of the present disclosure.
FIG. 6 is a graph showing results of evaluating platelet aggregation inhibitory effects of C-type natriuretic peptide analogues according to an embodiment of the present disclosure.
FIG. 7A is a graph showing changes in blood pressure after administering C-type natriuretic peptide analogues according to an embodiment of the present disclosure to rats.
FIG. 7B is a graph showing changes in heart rate after administering C-type natriuretic peptide analogues according to an embodiment of the present disclosure to rats.
FIG. 8 is a graph showing NEP resistance of C-type natriuretic peptide analogues according to an embodiment of the present disclosure to rats.
FIGS. 9A to 9C are graphs showing growth results of body weight (FIG. 9A), body length (FIG. 9B), and tail length (FIG. 9C) after administering C-type natriuretic peptide analogues according to an embodiment of the present disclosure to rats.
FIGS. 10A to 10C are graphs showing growth results of body weight (FIG. 10A), body length (FIG. 10B), and tail length (FIG. 10C) after administration and subsequent crossover administration of C-type natriuretic peptide analogues according to an embodiment of the present disclosure to rats.
FIG. 11A is a graph showing changes in body weight after administering C-type natriuretic peptide analogues according to an embodiment of the present disclosure at various doses.
FIG. 11B is a graph showing body length growth after administering C-type natriuretic peptide analogues according to an embodiment of the present disclosure at various doses.
FIG. 11C is a graph showing tail length growth after administering C-type natriuretic peptide analogues according to an embodiment of the present disclosure at various doses.
FIG. 11D is a photograph showing growth of mice after administering C-type natriuretic peptide analogues according to an embodiment of the present disclosure at various doses.

### [Best Mode]

The present disclosure relates to C-type natriuretic peptide analogues with increased half-life and bioavailability, and uses of the same for preventing, alleviating or treating growth disorders and promoting growth.

The C-type natriuretic peptide analogues of the present disclosure have advantages of being easy to synthesize peptide drugs and reducing production costs by exhibiting a prolonged *in vivo* half-life, resulting in high bioavailability, and a high bone growth rate, as well as having a relatively short length, and thus can be effectively used in compositions for promoting growth or in the treatment or prevention of bone-related diseases.

### (1) Half-life superiority

The half-life of a C-type natriuretic peptide analogue (CNP analogue) according to an embodiment of the present disclosure may be increased compared to a wild type, and may be increased by 1 to 200 times or more compared to the wild-type CNP, although the present disclosure is not limited thereto. Since such half-life superiority is an important factor in determining the administration frequency of peptide drugs, the CNP analogue may be advantageously used when developing peptide drugs.

### (2) Bioavailability (BA, %) superiority

The bioavailability of the C-type natriuretic peptide analogue (CNP analogue) according to an embodiment of the present disclosure may be increased compared to a wild-type CNP, and may be increased by 10 times or more compared to the wild type CNP, which is a control group, although the present disclosure is not limited thereto. Such an increase in bioavailability may be advantageously used in the development of drugs.

### (3) Superiority of activity and efficacy

When the C-type natriuretic peptide analogue (CNP analogue) according to an embodiment of the present disclosure is administered to a normal mouse, the bone growth rate may be at least twice as high as that of wild-type CNP for 1 to 2 weeks after administration. This may be due to the high bioavailability characteristic resulting from a prolonged half-life of the C-type natriuretic peptide analogue according to an embodiment of the present disclosure. Preferably, the C-type natriuretic peptide analogue according to an embodiment of the present disclosure may have enhanced activity and efficacy compared to the prior art.

Specifically, in the C-type natriuretic peptide analogue (CNP analogue) of the present disclosure, CNP analogue candidates may be prepared by introducing one or more mutations selected from the group consisting of substitution, deletion, and addition to amino acids constituting CNP to improve the efficacy of CNP. In the present disclosure, CNP analogue candidates with improved efficacy were selected by performing evaluation of resistance to neprilysin, as a neutral endopeptidase that degrades CNP to cause loss of function, pharmacokinetic evaluation to verify *in vivo* stability, evaluation of platelet aggregation inhibitory effect, evaluation of cardiovascular safety (hemodynamics), measurement of mouse growth, etc. However, any methods for evaluating activity and efficacy can be adopted by those skilled in the art, but the present disclosure is not limited thereto.

The C-type natriuretic peptide analogue according to an embodiment of the present disclosure includes one or more amino acid variations selected from the group consisting of one or more amino acid substitutions, one or more amino acid additions at the N-terminus and/or C-terminus, and one or more amino acid deletions of wild-type CNP. The C-type natriuretic peptide analogue according to an embodiment of the present disclosure may have an increased *in vivo* half-life due to increased stability while maintaining or increasing the physiological activity of wild-type CNP.

As an embodiment, the C-type natriuretic peptide analogue of the present disclosure may have a structure of the following Formula 1:

[Formula 1] (P1)-PPKKGPPNG-(P2)

In Formula 1, P1 is a peptide consisting of 0 to 4 amino acids and may include the following amino acid sequence.
P1 is (X1)-(X2)-(X3)-(X4),
X1 is Gly(G), Pro(P), Thr(T), Ala(A), Ser(S), or absent,
X2 is Asp(D), Gly(G), Lys(K), Ser(S), Asn(N), Glu(E), Gln(Q), or absent,
X3 is Asn(N), Lys(K), Asp(D), Ser(S), Gln(Q), or absent, and
X4 is Lys(K), Thr(T), Ser(S), Arg(R), Glu(E), Gln(Q), His(H), or absent.

In addition, more preferably, as an embodiment, in Formula 1, P1 is a peptide consisting of 0 to 4 amino acids and may include the following amino acid sequence.
P1 is (X1)-(X2)-(X3)-(X4),
X1 is Gly(G), Pro(P), or absent,
X2 is Asp(D), Gly(G), Ser(S), or absent,
X3 is Asn(N), Lys(K), or absent, and
X4 is Lys(K), Ser(S), or absent.

In addition, P2 is a peptide consisting of an amino acid sequence of SEQ ID NO: 2 or 3, or a peptide comprising one or more amino acid substitutions in the amino acid sequence of SEQ ID NO: 2 or 3.

Specifically, in Formula 1, P2 may be a peptide consisting of/comprising the amino acid sequence of SEQ ID NO: 2, a peptide comprising one or more amino acid substitutions in the amino acid sequence of SEQ ID NO: 2, or a peptide in which amino acids at positions 4, 6, and/or 12 in the amino acid sequence of SEQ ID NO: 2 are substituted.

For example, in Formula 1, P2 may be represented by the following Formula 2 (SEQ ID NO: 5):

[Formula 2] CFG-(X5)-K-(X6)-DRIGS-(X7)-SGLGC

In Formula 2,
X5 and X7 may be selected from amino acids having cationic or hydrophobic side chains, and X6 may be selected from amino acids having cationic or hydrophobic side chains excluding Ile(I).

As an example, in Formula 2, X5 may be Leu(L) or His(H), X6 may be Leu(L), and X7 may be Met(M) or His(H); specifically,
X5 may be Leu(L), X6 may be Leu(L), and X7 may be Met(M);
X5 may be Leu(L), X6 may be Leu(L), and X7 may be His(H);
X5 may be His(H), X6 may be Leu(L), and X7 may be Met(M); or
X5 may be His(H), X6 may be Leu(L), and X7 may be His(H), but the present disclosure is not limited thereto.

In addition, in Formula 2, the first cysteine (C) and the last cysteine (C) may form a ring via C-C bonds.

In the C-type natriuretic peptide analogue of the present disclosure, as a more preferred example,
P1 of Formula 1 may be (X1)-(X2)-(X3)-(X4),
X1 may be Gly(G), Pro(P), Thr(T), Ala(A), Ser(S), or absent,
X2 may be Asp(D), Gly(G), Lys(K), Ser(S), Asn(N), Glu(E), Gln(Q), or absent,
X3 may be Asn(N), Lys(K), Asp(D), Ser(S), Gln(Q), or absent, and
X4 may be Lys(K), Thr(T), Ser(S), Arg(R), Glu(E), Gln(Q), His(H), or absent, and simultaneously, P2 may be represented by the following Formula 2 (SEQ ID NO: 5):

   [Formula 2] CFG-(X5)-K-(X6)-DRIGS-(X7)-SGLGC

In Formula 2,
X5 and X7 may be selected from amino acids comprising cationic or hydrophobic side chains, and X6 may be selected from amino acids comprising cationic or hydrophobic side chains excluding Ile(I).

In a more specific embodiment of the present disclosure, in Formula 2, P2 may include an amino acid sequence in which
X5 may be Leu(L) or His(H),
X6 may be Leu(L), and X7 may be Met(M) or His(H), and
simultaneously, the P1 may be more specifically (X1)-(X2)-(X3)-(X4),
X1 is Gly(G), Pro(P), or absent,
X2 is Asp(D), Gly(G), Ser(S), or absent,
X3 is Asn(N), Lys(K), or absent, and
X4 is Lys(K), Ser(S), or absent.

The C-type natriuretic peptide analogue according to an embodiment of the present disclosure may include a substitution in which an acetyl group and/or fatty acid (e.g., 8-amino-3,6-dioxaoctanoic acid, miniPEG, miniPEG2, caprylic acid, capric acid, lauric acid, stearic acid, or palmitic acid, etc.) or a derivative thereof is introduced to at least one amino acid, a substitution in which some amino acids are substituted with Aminoisobutyric acid (Aib), a substitution in which some amino acids are substituted with D-type amino acids, etc.

The C-type natriuretic peptide analogue according to an embodiment of the present disclosure may have at least one acetylated amino acid in the amino acid sequence of Formula 1. For example, the C-type natriuretic peptide analogue according to an embodiment of the present disclosure may have an acetylated amino acid at the N-terminus.

The C-type natriuretic peptide analogue according to an embodiment of the present disclosure may be formed by introducing, directly or via a linker, a fatty acid or a derivative thereof, which is connected in one or more (e.g., 1, 2, or 3) repeating units, into at least one amino acid among first to fifth amino acids from the N-terminus, for example, a first amino acid from the N-terminus. In this case, the fatty acid may be a fatty acid having 3 to 21 carbon atoms, but is not limited thereto. Specifically, the fatty acid or the derivative thereof may be introduced in one or more repeating units into one or more of the first to fifth or first to fourth amino acids from the N-terminus of the C-type natriuretic peptide analogue. As an example, the fatty acid or the derivative thereof may be introduced into the first or third amino acid, or as an example, the fatty acid may be introduced into the first or third amino acid.

The fatty acid may be introduced into the C-type natriuretic peptide analogue according to an embodiment of the present disclosure to increase stability. For example, the fatty acid or the derivative thereof may include small PEGs (miniPEG) comprising a small polyethylene glycol moiety (PEG) having a structure [-O-CH₂-CH₂-]ₙ (wherein, n may be an integer of 2 to 16, e.g., 2, 3, 4, 5, 6, 7, 8, 9, 10, 11, 12, 13, 14, 15, or 16), functionalized miniPEG comprising one or more functional groups (comprising, but not limited to, amine, hydroxyl, thiol, and carboxyl groups, or any combination thereof), a fatty acid having 6 to 21 carbon atoms, a fatty acid having 13 to 21 carbon atoms, or derivatives of the fatty acids. As an example, the fatty acid or the derivative thereof may include 8-amino-3,6-dioxaoctanoic acid (trademark: miniPEG2), miniPEG, CH₃(CH₂)₁₄COOH (palmitic acid), caprylic acid, capric acid, lauric acid, stearic acid, etc.

The fatty acid or the derivative thereof may be introduced directly or via a linker into at least one amino acid in the amino acid sequence of the C-type natriuretic peptide analogue according to an embodiment of the present disclosure. For example, the linker may be gamma glutamic acid, aminocaproic acid (AHX), PEG, etc., but is not limited thereto.

The C-type natriuretic peptide analogue according to an embodiment of the present disclosure has the structure of Formula 1 and may include one or more substitutions selected from the group consisting of amino acid deletion, substitution, and addition. Specifically, the mutated CNP analogues according to an embodiment of the present disclosure may have amino acid sequences as described in Table 1 below. In Table 1 below, (C-C) indicates that cysteine and cysteine are bound to form a ring. Accordingly, the C-type natriuretic peptide analogue according to an embodiment of the present disclosure may include one or more amino acid sequences selected from the group consisting of SEQ ID NOs: 6 to 13.

**[Table 1]**

| SEQ ID NO: | Amino acid sequence |
|---|---|
| 6 | GDNKPPKKGPPNGCFGLKLDRIGSMSGLGC (C-C) |
| 7 | GDNKPPKKGPPNGCFGHKLDRIGSMSGLGC (C-C) |
| 8 | GDNKPPKKGPPNGCFGHKLDRIGSHSGLGC (C-C) |
| 9 | GDNKPPKKGPPNGCFGLKLDRIGSHSGLGC (C-C) |
| 10 | PGNKPPKKGPPNGCFGLKLDRIGSHSGLGC (C-C) |
| 11 | GD-K(gamma Glu-Pal)-KPPKKGPPNGCFGHKLDRIGSHSGLGC (C-C) |
| 12 | Palmitoyl-GDNKPPKKGPPNGCFGHKLDRIGSHSGLGC (C-C) |
| 13 | GS-K(gamma Glu-Pal)-SPPKKGPPNGCFGHKLDRIGSHSGLGC (C-C) |

The C-type natriuretic peptide analogue according to an embodiment of the present disclosure may improve one or more characteristics selected from the group consisting of *in vivo* half-life, bioavailability, and growth plate maintenance compared to wild-type CNP.

The C-type natriuretic peptide analogue according to an embodiment of the present disclosure may have a half-life of greater than 1 time, 1.5 times or more, 2 times or more, 2.5 times or more, 3 times or more, 3.5 times or more, 4 times or more, 4.5 times or more, 5 times or more, 5.5 times or more, 6 times or more, 6.5 times or more, 7 times or more, 7.5 times or more, 8 times or more, 8.5 times or more, 9 times or more, 9.5 times or more, 10 times or more, 15 times or more, 20 times or more, 25 times or more, 30 times or more, 35 times or more, 40 times or more, 45 times or more, 50 times or more, 55 times or more, or 60 times or more compared to the *in vivo* half-life of wild-type CNP. At this time, even if the upper limit value of the *in vivo* half-life is not specified, those skilled in the art will clearly understand the technical effect of the present disclosure, which has an improved *in vivo* half-life compared to wild-type CNP, but for example, the upper limit value of the *in vivo* half-life may be 200 times or less, 150 times or less, 100 times or less, 80 times or less, or 70 times or less than the *in vivo* half-life of wild-type CNP, but is not limited thereto.

In an embodiment, the C-type natriuretic peptide analogue according to an embodiment of the present disclosure may have an *in vivo* half-life of greater than 1 time, 1.5 times or more, 2 times or more, 2.5 times or more, 3 times or more, 3.5 times or more, 4 times or more, 4.5 times or more, 5 times or more, 5.5 times or more, or 6 times or more compared to BMN111. At this time, even if the upper limit value of the *in vivo* half-life is not specified, those skilled in the art will clearly understand the technical effect of the present disclosure, which has an improved *in vivo* half-life compared to BMN111, but for example, the upper limit value of the *in vivo* half-life may be 100 times or less, 50 times or less, 30 times or less, 20 times or less, 15 times or less, or 10 times or less of the *in vivo* half-life of BMN111, but is not limited thereto.

The bioavailability (BA) of the C-type natriuretic peptide analogue according to an embodiment of the present disclosure may be greater than 1 to 20 times or less, greater than 1 to 15 times or less, greater than 1 to 10 times or less, 2 to 20 times, 2 to 15 times, 2 to 10 times, 5 to 20 times, 5 to 15 times, or 5 to 10 times based on the bioavailability (BA) of wild-type CNP, and for example, the bioavailability (BA) of the C-type natriuretic peptide analogue according to an embodiment of the present disclosure may be increased by at least 2 times based on the bioavailability (BA) of wild-type CNP.

The C-type natriuretic peptide analogue according to an embodiment of the present disclosure may have a higher bone growth rate than wild-type CNP when administered in an amount equivalent to or less than that of wild-type CNP.

The C-type natriuretic peptide analogue according to an embodiment of the present disclosure may be characterized by having resistance to neutral endopeptidase, and specifically may have excellent neutral endopeptidase resistance compared to wild-type CNP and BMN111. For example, when the C-type natriuretic peptide analogue according to an embodiment of the present disclosure is treated with neutral endopeptidase, at least 88%, 89%, 90%, 91%, 92%, 93%, 94%, 95%, 96%, 97%, 98%, 99%, 99.5%, 99.9%, or 100% may remain after 200 minutes, and the C-type natriuretic peptide analogue according to an embodiment of the present disclosure may exhibit excellent neutral endopeptidase resistance compared to the control group.

In another aspect, the present disclosure relates to a composition for promoting growth or a composition for preventing or treating growth disorders, comprising the C-type natriuretic peptide analogue. The composition may be a pharmaceutical composition or a food composition.

The use for promoting growth may include, for example, increasing the growth rate and /or degree of growth of a subject. The target of growth promotion may be a normal subject or a subject with growth disorders, and preferably, a normal subject requiring growth promotion. Accordingly, an embodiment of the present disclosure relates to a composition for promoting growth comprising a C-type natriuretic peptide analogue of the present disclosure. The use for promoting growth may include increasing the growth rate and/or degree of growth of a subject, and may include, for example, one or more selected from the group consisting of maintaining the growth plate, expanding the growth plate, improving the shape of the growth plate, increasing bone length growth, and preventing and treating bone length growth disorders.

The composition for promoting growth may promote the growth of a subject when administered to the subject, and specifically, increase the growth rate of the subject or increase the degree of growth of the subject. The subject is not limited by age and may include, for example, both subjects that are growing (subjects in the growth phase) and subjects that have grown. For example, the composition for promoting growth may increase the degree of growth by promoting the growth rate of the subject that is growing, or increase the growth of the subject that has grown.

The increase in growth rate or degree of growth of the subject may be evaluated by measuring variables such as height, sitting height, body weight, head circumference, upper arm length, lower arm length, upper leg length, lower leg length, hand length, and/or foot length, but is not limited thereto.

The increase in the growth rate of the subject may be an increased growth rate compared to the growth rate of the subject before administration of the composition for promoting growth according to an embodiment of the present disclosure, or a baseline growth rate. The baseline may be determined from a group of subjects having the same general age, gender, and disease condition as the subject. For example, the increase in the growth rate of the subject may be an increase in annual growth rate in the change range of 5% to 50% from the baseline of the subject, and specifically, the composition for promoting growth according to an embodiment of the present disclosure may have an annual increase in the growth rate measured by the evaluation of the variable (e.g., height, etc.) of at least about 5%, 10%, 15%, 20%, 25%, 30%, 35%, 40%, 45%, or 50%, or more than the baseline, and may cause an annual increase in the growth rate measured by height of at least about 5%, 10%, 15%, 20%, 25%, 30%, 35%, 40%, 45%, or 50% or more than the baseline. Alternatively, the composition may cause an annual increase in growth rate measured by sitting height, body weight, head circumference, upper arm length, lower arm length, upper leg length, lower leg length (from knee to foot), hand length (from wrist to fingertip) or foot length of at least about 5%, 10%, 15%, 20%, 25%, 30%, 35%, 40%, 45%, or 50% or more than the baseline. The baseline may be an annualized growth rate measured by specific variables before administering the CNP according to the present disclosure to the same subject, or a growth rate measured from a subject group having the same general age, gender, and disease condition as the subject that is administered with the CNP according to the present disclosure.

Yet another embodiment of the present disclosure relates to a composition for preventing or treating growth disorders comprising a C-type natriuretic peptide analogue of the present disclosure, and an example of the growth disorders may be bone-related diseases or bone dysplasia. The composition may be a pharmaceutical composition or a food composition. The use for preventing or treating growth disorders includes, for example, alleviation, improvement, treatment, or prevention of growth disorders in a subject. The target for the prevention or treatment may be a subject having growth disorders, preferably a subject having bone-related diseases or disorders.

The bone-related diseases or bone dysplasias may be one or more selected from the group consisting of achondroplasia, osteoarthritis, hypophosphatemic rickets, hypochondroplasia, short stature, dwarfism, osteochondrodysplasias, thanatophoric dysplasia, osteoogenesis imperfecta, achondrogenesis, chondrodysplasia punctata, homozygous achondroplasia, chondrodysplasia punctata, camptomelic dysplasia, congenital lethal hypophosphatasia, perinatal lethal type of osteogenesis imperfecta, short-rib polydactyly syndromes, hypochondroplasia, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, aromesomelic dysplasia, micromelia, Morquio syndrome, Kniest syndrome, metatrophic dysplasia, spondyloepimetaphyseal dysplasia, and Noonan syndrome.

In the present disclosure, the "analogues" are peptide analogues, in which one or more amino acids may be substituted, deleted, inverted, or added compared to a wild type, and may include extension and/or cleavage at the N-terminal or C-terminal position, and are one or more modified amino acids, which may include glycosylated amino acids, PEGylated amino acids, farnesylated amino acids, acetylated amino acids, acylated amino acids, biotinylated amino acids, non-natural amino acids, amino acids conjugated to lipid moieties such as fatty acids, or amino acids conjugated to organic derivatization. In addition, the analogues may include derivatives or substitutions of the wild-type peptide.

The "treatment" refers to treatment for preventive purpose, treatment for therapeutic purposes, or treatment for diagnostic purposes, etc., and in specific embodiments, the "treatment" refers to administering a composition to a subject for therapeutic purpose, preventive purpose, or diagnostic purpose, etc.

A therapeutic agent for "preventive purpose" is a therapeutic agent administered to a subject who does not show signs of diseases or shows only early signs of diseases, for the purpose of reducing a risk of the development of a pathological condition. The composition according to an embodiment of the present disclosure may be provided as a preventive therapeutic agent to reduce a possibility of a pathological condition, or to minimize the severity of the pathological condition if developed.

A therapeutic agent for "therapeutic purpose" is a therapeutic agent administered to a subject exhibiting signs or symptoms of a pathological condition for the purpose of reducing or eliminating the signs or symptoms of the pathological condition. The signs or symptoms may be biochemical, cellular, histological, functional, or physical, subjective, or objective. In addition, the composition of the present disclosure may be provided as a therapeutic agent for therapeutic purposes or for diagnosis.

The "diagnosis" means confirming the existence, range, and/or nature of a pathological condition. Diagnostic methods differ in specificity and selectivity. Even if a specific diagnostic method may not provide a clear diagnosis of a certain condition, it is sufficient if the method provides clear signs that aid in diagnosis.

Yet another embodiment of the present disclosure relates to a pharmaceutical composition comprising a C-type natriuretic peptide analogue according to an embodiment of the present disclosure.

The "pharmaceutical composition" refers to a composition suitable for pharmaceutical use in target animals, including humans and animals. The pharmaceutical composition includes a therapeutically effective amount of a CNP analogues, optionally another biologically active agent, and optionally a pharmaceutically acceptable excipient, carrier, or diluent. In an embodiment, the pharmaceutical composition includes active ingredient(s) and inactive ingredient(s) consisting of a carrier, as well as any product indirectly or directly formed from the combination, complexation, or aggregation of any two or more of these raw materials, or formed from one or more other types of reactions or interactions of these raw materials. Accordingly, the pharmaceutical composition according to an embodiment of the present disclosure includes any composition prepared by mixing the C-type natriuretic peptide analogue according to an embodiment of the present disclosure with a pharmaceutically acceptable excipient, carrier, or diluent.

The "pharmaceutically acceptable carrier" refers to standard pharmaceutical carriers, buffers, etc., and for example, includes a phosphate-buffered saline solution, a 5% aqueous dextrose solution, and an emulsion (e.g., oil/water or water/oil emulsions). Non-limiting examples of the excipient include adjuvants, binders, fillers, diluents, disintegrants, emulsifiers, wetting agents, lubricants, slip modifiers, sweeteners, flavoring agents, and coloring agents. The desirable pharmaceutical carrier depends on an intended administration method of the active agent. Typical administration methods include rectal (e.g., oral) or parenteral (e.g., subcutaneous, intramuscular, intravenous, or intraperitoneal injection; or topical, transdermal, or transmucosal) administration.

The "pharmaceutically acceptable salt" refers to salts that may be formulated into compositions for pharmaceutical use by including metal salts (e.g., sodium, potassium, magnesium, calcium, etc.) and ammonia or organic amine salts, but is not limited thereto.

The "pharmaceutically acceptable" or "pharmacologically acceptable" means that a substance that is biologically or otherwise undesirable, that is, this substance may be administered to a subject without causing any undesirable biological effects or interacting in a harmful manner with ingredients in a composition containing the substance or ingredients present on or within the subject's body.

The term "subject" includes mammals and non-mammals. Examples of the mammals include, but are not limited to, members of the mammalian class, namely humans, mammals, primates, primates other than humans, mammals other than humans, such as chimpanzee, monkey, and chimpanzee species; livestock such as cattle, horses, sheep, goats, and pigs; pets such as rabbits, dogs, and cats; and laboratory animals including rodents, such as rats, mice, and guinea pigs. Examples of the non-mammals include, but are not limited to, birds, fish, etc.

The pharmaceutical composition may optionally further include other biological active agents, optionally pharmaceutically acceptable excipients, carriers, or diluents.

Yet another embodiment of the present disclosure relates to a food composition comprising a C-type natriuretic peptide analogue according to an embodiment of the present disclosure.

The "food composition" may be a functional food or a health functional food, and particularly, the "health functional food" refers to foods produced and processed using raw materials or ingredients that have functionality useful to the human body, and the "functionality" means consuming for the purpose of obtaining useful effects for health purposes, such as regulating nutrients or physiological actions on the structure and function of the human body.

The food or health functional food of the present disclosure may be produced and processed into pharmaceutical administration forms such as powders, granules, tablets, capsules, pills, suspensions, emulsions, syrups, etc., or into functional foods such as tea bags, leached tea, beverages, candies, jellies, and gums for the purpose of promoting growth. Yet another embodiment of the present disclosure relates to a method for promoting growth, a method for preventing growth disorders, or a method for treating growth disorders, comprising administering a C-type natriuretic peptide analogue of the present disclosure. The C-type natriuretic peptide analogue of the present disclosure is as described above. The method for promoting growth, the method for preventing growth disorders, or the method for treating growth disorders of the present disclosure may include administering the C-type natriuretic peptide analogue of the present disclosure into a subject, and the method of administration may include any conventional method in the art without limitation. For example, the C-type natriuretic peptide analogue of the present disclosure may be administered to a subject according to known administration methods, either orally or parenterally, and preferably administered parenterally, such as intravenously, subcutaneously, intraperitoneally, or intraarticularly, such as endochondrally. In addition, the C-type natriuretic peptide analogue may be administered to a normal subject or a subject with growth disorders, and preferably to a normal subject in need of growth promotion. The growth promotion, growth disorders, etc., are as described above.

The C-type natriuretic peptide analogue according to an embodiment of the present disclosure may be administered according to standards known in the art, depending on a condition of each patient. For example, the daily dose may be 0.01 to 500 nmol/kg, but is not limited thereto. In addition, the daily dose may vary depending on various factors such as the age, health condition, and complications of a subject to be administered, and the C-type natriuretic peptide analogue according to an embodiment of the present disclosure has a prolonged *in vivo* half-life compared to a conventional C-type natriuretic peptide, and thus may be administered at a lower dose or with fewer number of administration times than the conventional C-type natriuretic peptide.

Hereinafter, in order to specifically describe the present specification, Examples will be described in detail. However, Examples according to the present specification may be modified in various other forms, and the scope of the present specification is not to be interpreted as being limited to Examples to be described below. Examples of the present specification are provided to more fully explain the present specification to those skilled in the art.

### [Modes]

### Example 1. Preparation of novel CNP analogues for enhanced stability in blood

In order to enhance stability in blood, novel CNP analogues represented by SEQ ID NOs: 6 to 10 were prepared by introducing a peptide sequence containing the amino acid sequence of SEQ ID NO: 1 (PPKKGPPNG) into variously modified CNP sequences. In addition, the half-lives thereof were measured, and the results were shown in Table 2.

**[Table 2]**

| Classification | Amino acid sequence | MW (Cal./Found) | Retention time (min) | Half-life (min) |
|---|---|---|---|---|
| Control wild-type CNP (SEQ ID NO: 3) | GLSKGCFGLKLDRIGSMSGLGC (C-C) | 2,196/2,197 | 5.925 | N/A |
| SEQ ID NO: 6 | GDNKPPKKGPPNGCFGLKLDRIGSMSGLGC (C-C) | - | N/A | 12.71 |
| SEQ ID NO: 7 | GDNKPPKKGPPNGCFGHKLDRIGSMSGLGC (C-C) | 3,066/3,066 | 6.500 | 15.91 |
| SEQ ID NO: 8 | GDNKPPKKGPPNGCFGHKLDRIGSHSGLGC (C-C) | 3,071/3,072 | 6.017 | 15.95 |
| SEQ ID NO: 9 | GDNKPPKKGPPNGCFGLKLDRIGSHSGLGC (C-C) | 3,047/3,048 | 6.667 | 14.24 |
| SEQ ID NO: 10 | PGNKPPKKGPPNGCFGLKLDRIGSHSGLGC (C-C) | 3,029/3,030 | 6.575 | 12.57 |

The novel CNP analogues prepared according to the present disclosure exhibited a half-life approximately 10 times longer than the half-life of 1.6 minutes of the control wild-type CNP.

This means that the half-lives of the CNP analogues prepared in the present disclosure increased and the stability in blood was increased by the addition of the amino acid sequence of SEQ ID NO: 1.

### Example 2. Pharmacokinetic evaluation of novel CNP analogues (1): Determination of half-life

In order to evaluate the blood stability of the novel CNP analogues of SEQ ID NOs: 6 to 9 prepared in Example 1, a pharmacokinetic evaluation was performed.

Specifically, the peptides of SEQ ID NOs: 6 to 9 were administered intravenously to rats once at a concentration of 50 nmol/kg. As a control group (Comparative Example), wild-type CNP of SEQ ID NO: 3 without the amino acid sequence of SEQ ID NO: 1 was used. In addition, BMN111 (SEQ ID NO: 14) was used as a positive control group.

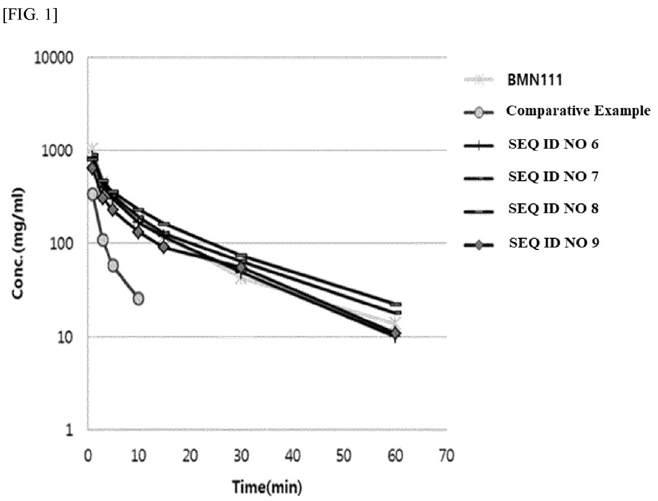

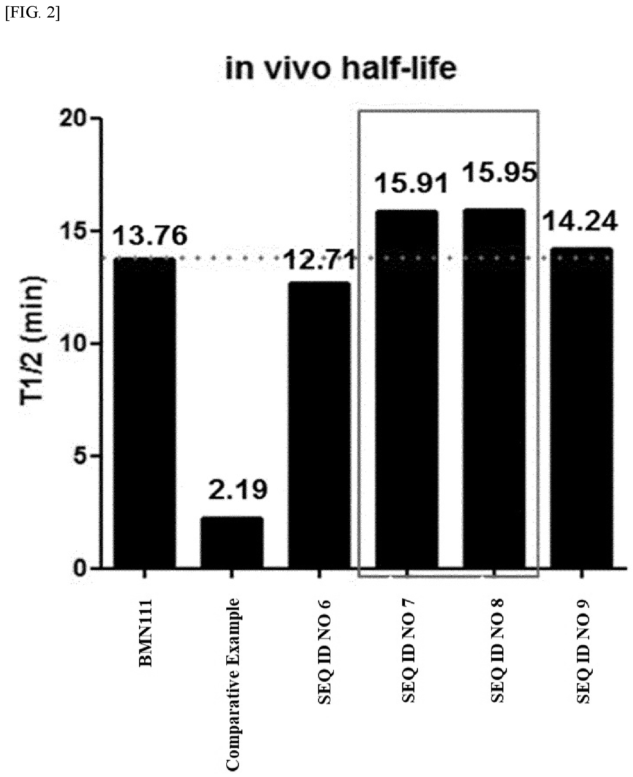

Blood was collected at 1, 3, 5, 10, 15, 30, and 60 minutes after administration, plasma was isolated, and then the amounts of candidates remaining in intact forms were measured using LC-MS/MS. The measurement results were shown in FIG. 1, and based on the measurement results, the half-lives were calculated and shown in FIG. 2.

As shown in FIG. 1 and 2, compared to the control group (Comparative Example), the half-life of BMN111 (SEQ ID NO: 14) was 13.7 minutes, and the half-lives of the novel CNP analogue peptides of SEQ ID NOs: 6, 7, 8, and 9 were 12.71 minutes, 15.91 minutes, 15.95 minutes, and 14.24 minutes, respectively. In particular, the peptides of SEQ ID NOs: 7 and 8, in which a sequence at position 17 was substituted with 'H (histidine)', had half-lives increased by approximately 10% or more compared to BMN111, thereby confirming that the novel CNP analogues, into which a peptide containing the amino acid sequence of SEQ ID NO: 1 had been introduced into the CNP sequence, were more stable in blood.

From these results, the peptides of SEQ ID NOs: 7 and 8 with a more improved half-life than BMN111 were selected as candidates for subsequent experiments, and in particular, the peptide of SEQ ID NO: 8, which had 'H (histidine)' introduced at position 25, was selected as a representative candidate for subsequent experiments.

### Example 3. Derivation of novel CNP analogues for enhanced blood stability (2): Introduction of palmitoyl group

Next, in order to further enhance stability in blood, novel CNP analogues were synthesized by introducing a palmitoyl group into the peptide of SEQ ID NO: 8, as a candidate from the results of Example 2, and a peptide having substitution or deletion of 0 to 4 amino acids at the N-terminus of the sequence. Subsequently, EC50 values, which were half maximal effective concentrations of the candidates, were measured using GraphPad Prism software.

**[Table 3]**

| Classification | Amino acid sequence | MW (Cal./Found) | Retention time (min) | EC₅₀ (nM) |
|---|---|---|---|---|
| Wild type CNP (SEQ ID NO: 3) | GLSKGCFGLKLDRIGSMSGLGC (C-C) | 2,196/2,197 | 5.925 | 68.8 |
| SEQ ID NO: 11 | GD-K(gamma Glu-Pal)- KPPKKGPPNGCFGHKLDRIGSHSGLGC (C- C) | 3,454/3,454 | 6.642 | 0.3 |
| SEQ ID NO: 12 | Palmitoyl- GDNKPPKKGPPNGCFGHKLDRIGSHSGLGC (C-C) | 3,308/3,309 | 6.467 | 0.5 |
| SEQ ID NO: 13 | GS-K(gamma Glu-Pal)- SPPKKGPPNGCFGHKLDRIGSHSGLGC (C- C) | 3,383/3,384 | 6.808 | 0.1 |

As shown in Table 3, when the EC₅₀ values were calculated, the wild-type CNP showed the EC₅₀ value of 68 nM, but the peptides of SEQ ID NOs: 11, 12, and 13, which were candidates with palmitoyl groups introduced to enhance stability in blood, showed the EC₅₀ values of 0.3 nM, 0.5 nM, and 0.1 nM, respectively.

This result indicates that the novel CNP analogues according to the present disclosure were substances in which the EC₅₀ values were significantly improved by approximately 150 to 700 times higher than the wild-type CNP.

### Example 4. Pharmacokinetic evaluation of novel CNP analogues (2): Intravenous administration

In order to evaluate the blood stability of the novel CNP analogue (palmitoyl-CNP analogue) prepared in Example 3, a pharmacokinetic (PK) evaluation was performed by intravenous administration.

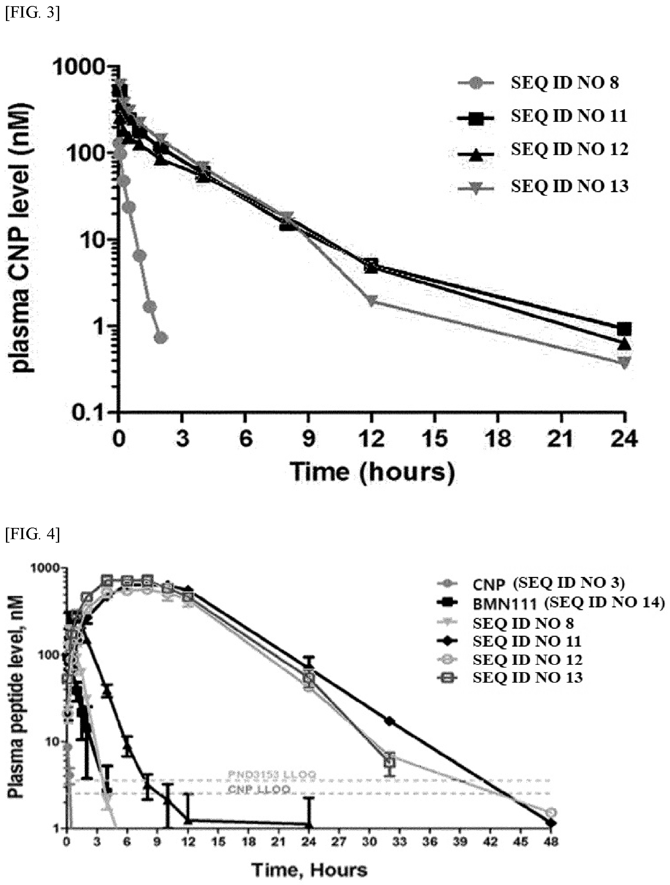

Specifically, the peptides of SEQ ID NOs: 8, 11, 12, and 13 prepared in Examples 1 and 3 were administered intravenously once to SD rats (8 weeks old, 5 rats) at a concentration of 50 nmol/kg. Blood was collected up to 24 hours after administration, plasma was isolated, and then the amounts of candidates remaining in their intact forms were measured using LC-MS/MS, and the results were shown in Table 4 and FIG. 3.

**[Table 4]**

| Classification | Cₘₐₓ | T_{1/2} | AUC all |
|---|---|---|---|
| | (nM/L) | (min) | (min*nM/L) |
| SEQ ID NO: 8 | 240.0 | 17.1 | 2619.8 |
| SEQ ID NO: 11 | 2232.2 | 129.5 | 52208.8 |
| SEQ ID NO: 12 | 1873.7 | 179.5 | 40794.7 |
| SEQ ID NO: 13 | 3132.4 | 145.3 | 61988.2 |

As shown in Table 4 and FIG. 3, it was determined that the half-lives of the peptides of SEQ ID NOs: 11, 12, and 13, to which the palmitoyl group was introduced, increased by approximately 7.6 to 10 times or more compared to the peptide of SEQ ID NO: 8, to which the palmitoyl group was not introduced, and the area under the curve (AUC) increased by approximately 15 to 23 times or more.

As a result, it can be seen that when the C-type natriuretic peptide analogue according to the present disclosure was administered intravenously, the half-life was significantly increased and bioavailability was excellently improved compared to wild-type CNP by addition, substitution and/or deletion of amino acids, and selective introduction of fatty acids to wild-type CNP.

### Example 5. Pharmacokinetic evaluation of novel CNP analogues (3): Subcutaneous administration

Next, in order to determine whether the novel CNP analogue with blood stability confirmed in Example 4 was a drug that can be administered subcutaneously, a pharmacokinetic evaluation was performed by subcutaneous administration.

Specifically, the peptides of SEQ ID NOs: 8, 11, 12, and 13 prepared in Examples 1 and 4 were administered subcutaneously once to SD rats (8 weeks old, 5 rats) at a concentration of 50 nmol/kg. Blood was collected up to 48 hours after administration,plasma was isolated, and then the amounts of candidates remaining in their intact forms were measured using LC-MS/MS, and the results were shown in Table 5 and FIG. 4.

**[Table 5]**

| PK parameter | Cₘₐₓ (pmol/ml) | Tₘₐₓ (min) | AUCₗₐₛₜ (pmol*min/ml) | T_{1/2} (min) | BA (%) |
|---|---|---|---|---|---|
| Wild type CNP (SEQ ID NO: 3) | 8.7(1.0) | 5.0(0.0) | | - | 3.2 |
| BMN111(SEQ ID NO: 14) | 95.6(41.0) | 5.0(0.0) | 6004.1(1345.0) | 38.3(25.3) | 14.0 |
| SEQ ID NO: 8 | 171.2(61.2) | 10.0(7.1) | 11210.5(4344.7) | 47.7(27.2) | 25.4 |
| SEQ ID NO: 11 | 657.2(40.9) | 480.0(169.7) | 527103.4(62238.6) | 246.9 | 110.3 |
| SEQ ID NO: 12 | 607.4(4..3) | 360.0(169.7) | 456278.5(31788.2) | 202.5(14.4) | 127.8 |
| SEQ ID NO: 13 | 727.0(137.9) | 300.0(84.9) | 564614.3(41880.6) | 200.3(24.1) | 92.5 |

As shown in Table 5 and FIG. 4, the pharmacokinetic evaluation results of the control wild-type CNP and BMN111 were similar to the results of previous studies, to demonstrating the reliability of this experiment.

In the case of the peptide of SEQ ID NO: 8 without the palmitoyl group introduced, the half-life was 47.7 minutes, which was approximately 1.2 times longer than the half-life of the positive control group BMN111 of 38.3 minutes, and accordingly, the area under the curve increased approximately 1.8 times or more.

In addition, it was determined that the peptides of SEQ ID NOs: 11, 12, and 13, to which the palmitoyl group was introduced, had half-lives increased by approximately 5 to 6 times compared to the positive control group BMN111, and the area under the curve was significantly increased approximately 75 to 94 times or more.

Through these results, it was found that even when administered subcutaneously, the C-type natriuretic peptide analogue of the present disclosure showed a significantly increased half-life compared to wild-type CNP as well as the positive control group BMN111, like when administered intravenously. This shows that the CNP analogue of the present disclosure can be administered to a subject in various forms, including intravenous and subcutaneous administration.

### Example 6. Bioavailability (BA) analysis of novel CNP analogues

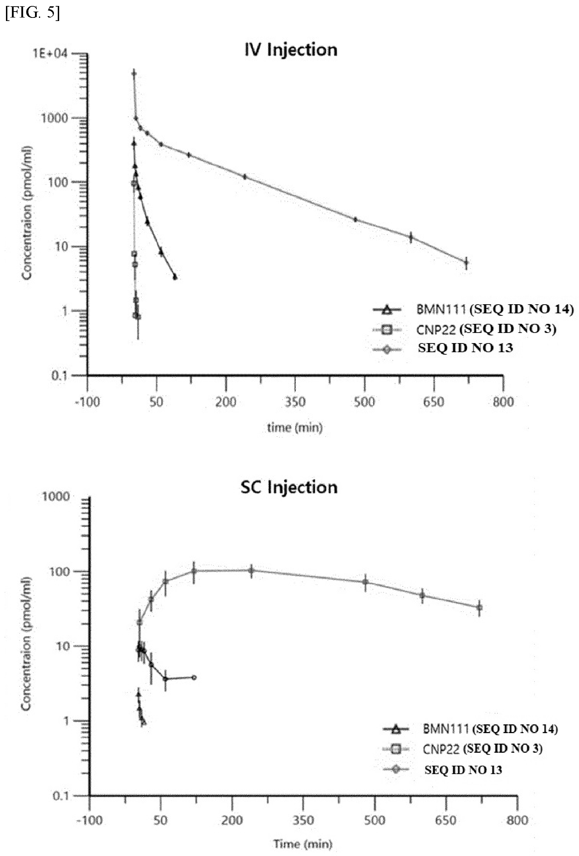

In order to confirm the bioavailability of the novel CNP analogues prepared in Examples 1 and 3 above, the bioavailability was analyzed by intravenous administration (IV) and subcutaneous administration (SC) at a dose of 70.0 nmol/kg with reference to Examples 4 and 5. The results were shown in Table 6 and FIG. 5.

**[Table 6]**

| PK parameter | Dose | Administration method | Cₘₐₓ | Tₘₐₓ | AUCₗₐₛₜ | T_{1/2} | BA_{abs} |
|---|---|---|---|---|---|---|---|
| PK parameters | nmol/kg | | pmol/ml | min | pmol*min/ml | min | % |
| Wild type CNP (SEQ ID NO: 3) | 70.0 | IV | 95.06 ± 24.40 | 1.00 ± 0.00 | 290.47 ± 80.01 | 1.59 ± 0.78 | - |
| BMN111(SEQ ID NO: 14) | 70.0 | IV | 410.53 ± 84.99 | 1.00 ± 0.00 | 3476.37 ± 255.58 | 17.89 ± 1.45 | - |
| SEQ ID NO: 13 | 70.0 | IV | 4809.40 ± 841.16 | 1.00 ± 0.00 | 108194.93 ± 5936.89 | 109.24 ± 3.94 | - |
| Wild type CNP (SEQ ID NO: 3) | 70.0 | SC | 2.28 ± 0.52 | 3.00 ± 0.00 | 14.49 ± 3.96 | - | 4.99 ± 1.36 |
| BMN111 (SEQ ID NO: 14) | 70.0 | SC | 10.31 ± 2.83 | 8.60 ± 5.90 | 341.78 ± 253.11 | 38.10 ± 24.68 | 9.83 ± 7.28 |
| SEQ ID NO: 13 | 70.0 | SC | 110.58 ± 28.66 | 192.00 ± 65.73 | 52803.82 ± 10118.05 | 224.30 ±26.62 | 48.80 ± 9.35 |

As shown in Table 6 and FIG. 5, when administered as a single intravenous dose of 70 nmol/kg, the *in vivo* half-life of the CNP analogue of SEQ ID NO: 13 was measured to be approximately 109.2 minutes, which was significantly increased compared to the half-life of the wild-type CNP of SEQ ID NO: 3, which was approximately 1.6 minutes, and the half-life of BMN111, which was approximately 18 minutes.

In addition, for the bioavailability, when administered as a single subcutaneous dose of 70 nmol/kg, the bioavailability of the positive control group BMN111 was found to be approximately 10%, while the bioavailability of the CNP analogue of SEQ ID NO: 13 was found to be approximately 48%. This means that the novel CNP analogue of SEQ ID NO: 13 according to the present disclosure exhibited an excellent physiological activity effect *in vivo*, with bioavailability increased approximately 4 times or more compared to BMN111.

### Example 7. Evaluation of platelet aggregation inhibitory effect of novel CNP analogues

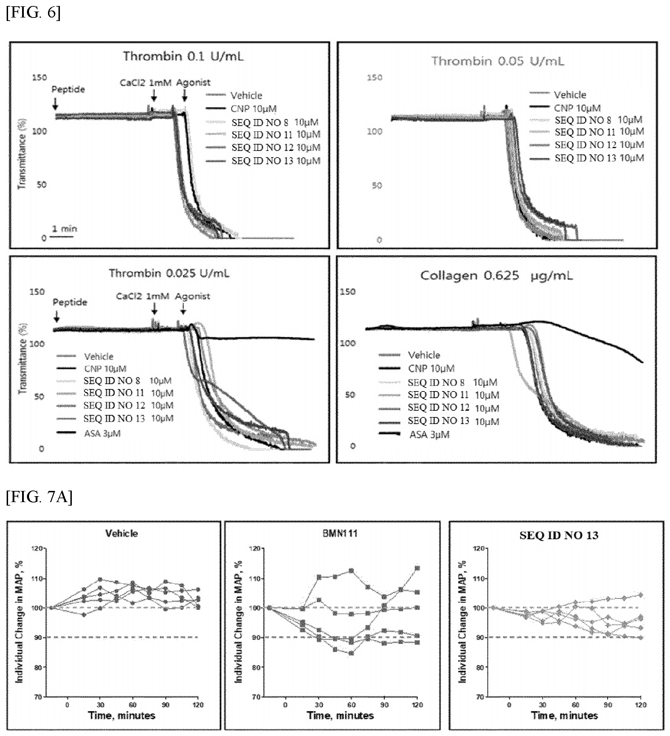

To determine whether the novel CNP analogues had side effects related to platelet aggregation, SD rat platelets were first isolated and then treated with agonists, collagen and thrombin to induce platelet aggregation. Next, platelet aggregation activity was observed using a light transmission aggregometer after treatment with CNP analogues of SEQ ID NOs: 8, 11, 12, and 13, and the results of the observation were shown in FIG. 6.

As shown in FIG. 6, as a result of confirming the platelet aggregation reaction induced by collagen and thrombin at a concentration of 10 µM, which was at least 20 times higher than the usage concentration of the CNP analogues according to an embodiment of the present disclosure, the CNP analogues according to an embodiment of the present disclosure did not cause platelet aggregation to a significant level. This means that the CNP analogues according to the present disclosure are substances without side effects on platelet aggregation.

### Example 8. Evaluation of cardiovascular safety (hemodynamics) of novel CNP analogues

To evaluate the cardiovascular safety of CNP analogues according to one example of the present disclosure, blood pressure and heart rate were measured.

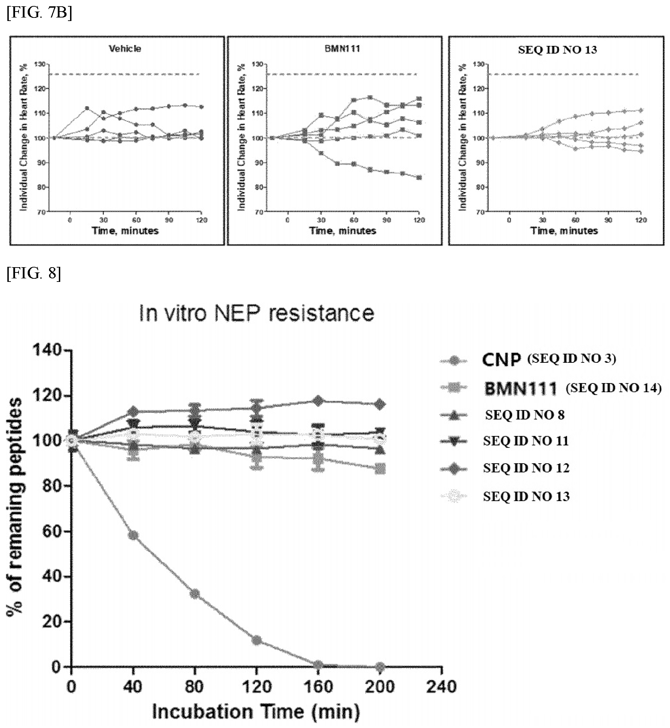

Specifically, the peptide of SEQ ID NO: 13, a CNP analogue according to an embodiment of the present disclosure, was administered subcutaneously once to about 5 rats (n = 5) at a concentration of 200 nmol/kg. Thereafter, blood was collected from 0 to 120 minutes, and then blood pressure change (MAP change) and heart rate for each subject were measured. The blood pressure change was shown in FIG. 7A, and the heart rate change was shown in FIG. 7B.

According to FIGS. 7A and 7B, the CNP analogue of SEQ ID NO: 13 showed less and more constant changes in blood pressure and heart rate for each subject than those of the positive control group BMN111, confirming that it was a substance with cardiovascular safety.

### Example 9. Evaluation of neutral endopeptidase (NEP) resistance of novel CNP analogues

In order to evaluate the stability of CNP analogues according to an embodiment of the present disclosure, resistance to *in vitro* cleavage by neutral endopeptidase (NEP) was confirmed.

CNP (SEQ ID NO: 3), BMN111 (SEQ ID NO: 14), and CNP analogues of SEQ ID NOs: 8, 11, 12, and 13 were prepared at a concentration of 4 µM using PBS containing 1% BSA, and 400 ng/ml of the neutral endopeptidase neprilysin was mixed in a 1 : 1 (v/v) ratio. While placed in a 37°C water bath for 0, 40, 80, 120, 160, and 200 minutes, each sample was added with 10 mM EDTA, and DTT and EDTA, and then put in ice to stop the reaction. Each sample was analyzed by LC-MS/MS after adding 1/10 (v/v) of 0.1% FA and 25% acetonitrile (ACN) (organic solvent for LC-MS/MS analysis).

As a result, as shown in Table 7 and FIG. 8 below, it was determined that the control group CNP was completely degraded after 160 minutes, and approximately 88% of BMN111 remained, but it was confirmed that the CNP analogues of SEQ ID NOs: 8, 11, 12 and 13 according to an embodiment of the present disclosure had high NEP resistance compared to the control groups.

**[Table 7]**

| Classification | Molecular weight | NEP resistance |
|---|---|---|
| | kDa | % Intact |
| Wild type CNP (SEQ ID NO: 3) | 2.2 | 0.0 |
| BMN111 (SEQ ID NO: 14) | 4.1 | 87.5 ± 2.6 |
| SEQ ID NO: 8 | 3.0 | 96.5 ± 2.4 |
| SEQ ID NO: 11 | 3.4 | 103.5 ± 2.9 |
| SEQ ID NO: 12 | 3.3 | 100.7 ± 0.7 |
| SEQ ID NO: 13 | 3.3 | 116.0 ± 1.1 |

### Example 10. Evaluation of growth plate maintenance in normal mice (1)

The growth plate maintenance effect in normal mice was evaluated using the CNP analogue of SEQ ID NO: 13, which had an excellent half-life among the CNP analogues prepared in Example 3.

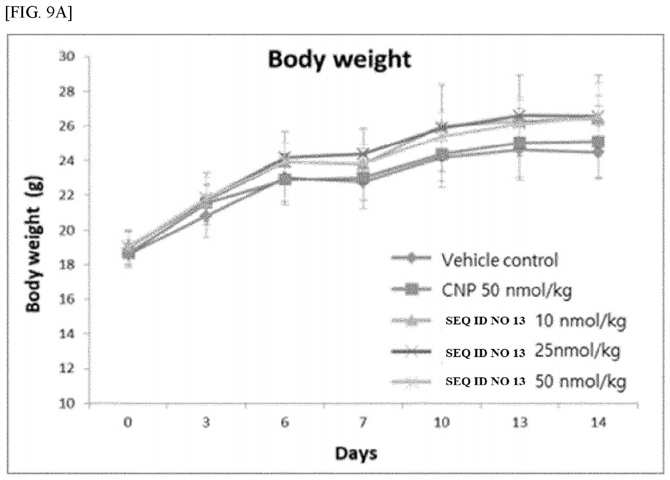

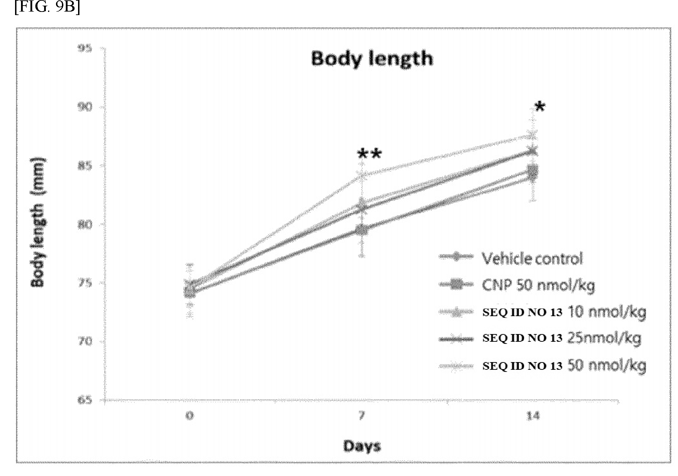

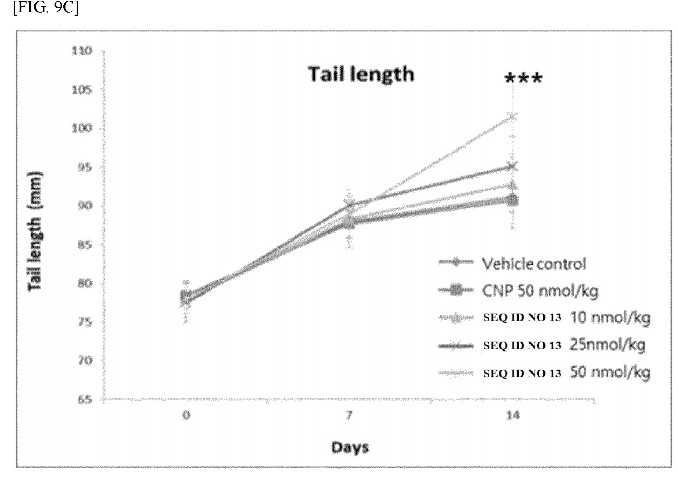

Specifically, as shown in Table 8, wild-type CNP and the CNP analogue of SEQ ID NO: 13 were each dissolved in an excipient (5% mannitol in saline), and then 3-week-old female ICR mice were subcutaneously injected daily with vehicle, CNP (50 nmol/kg), and the peptide of SEQ ID NO: 13 (10, 25, 50 nmol/kg) for 2 weeks, respectively, and the body weights, body lengths, and tail lengths of mice according to drug administration were measured and shown in Table 9 and FIGS. 9A to 9C.

**[Table 8]**

| | Vehicle control | Con(-) | Case 1 | Case 2 | Case 3 |
|---|---|---|---|---|---|
| Drug | 5% Mannitol | Wild type CNP (SEQ ID NO: 3) | SEQ ID NO: 13 | | |
| Dose (mg/kg) | | 50 nmol/kg | 10 nmol/kg | 25 nmol/kg | 50 nmol/kg |
| Injection volume | 5 ml/kg | 5 ml/kg | 5 ml/kg | 5 ml/kg | 5 ml/kg |
| Route of administration | SC,QD | SC,QD | SC,QD | SC,QD | SC,QD |
| (method of administration) | | | | | |
| Number of animals (N=) | 8 | 6 | 6 | 8 | 8 |

**[Table 9]**

| | Dose (nmol/kg/day) | Changes in body weight and body length | | |
|---|---|---|---|---|
| | | Body weight (g) | Body length (mm) | Tail length (mm) |
| Vehicle | 0 | 27.14 ± 2.46 | 86.88 ± 2.9 | 94.8 ± 3.28 |
| CNP (SEQ ID NO: 3) | 50 | 26.8 ± 1.95 | 86.5 ± 1.97 | 93.3 ± 1.86 |
| SEQ ID NO: 13 | 50 | 28.06 ± 2.28 | 89.75 ± 2.25 | 104.5 ± 4.56 |
| | 25 | 28.11 ± 2.56 | 88.4 ± 3.24 | 98.6 ± 3.66 |
| | 10 | 28.95 ± 0.78 | 89.8 ± 3.87 | 96.8 ± 3.25 |

As shown in Table 9 and FIGS. 9A to 9C, when the CNP analogues according to an embodiment of the present disclosure were administered to mice, the body weight, body length, and tail length showed concentration-dependent growth. In particular, it was determined that the body length (mm) of normal mice increased by approximately 2.9 mm compared to vehicle and by approximately 3.3 mm compared to wild-type CNP, and the tail length (mm) increased by approximately 9.7 mm compared to vehicle and by approximately 11.2 mm compared to wild-type CNP. In other words, it can be seen that when the CNP analogue of the present disclosure was administered in an amount equal to or smaller than that of wild-type CNP, the bone growth rate of mice was significantly increased.

### Example 11. Evaluation of mouse growth plate maintenance by cross-administration (2)

Growth plate maintenance in normal mice and growth plate maintenance by crossover administration were evaluated using the peptide of SEQ ID NO: 13, which had an excellent half-life among the CNP analogues prepared in Example 3.

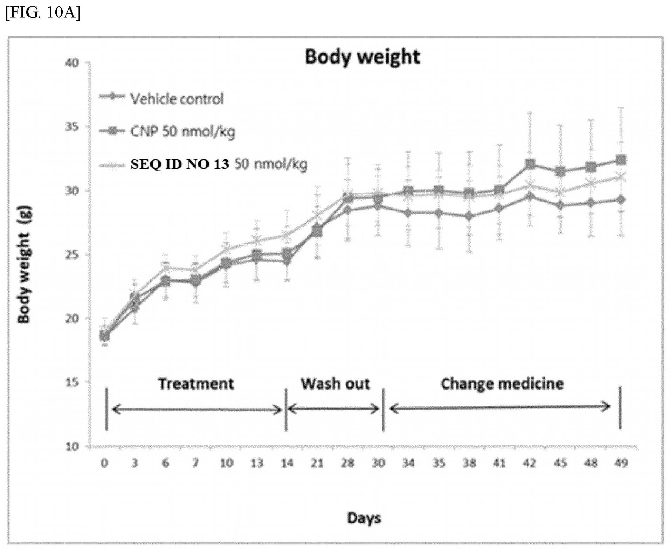

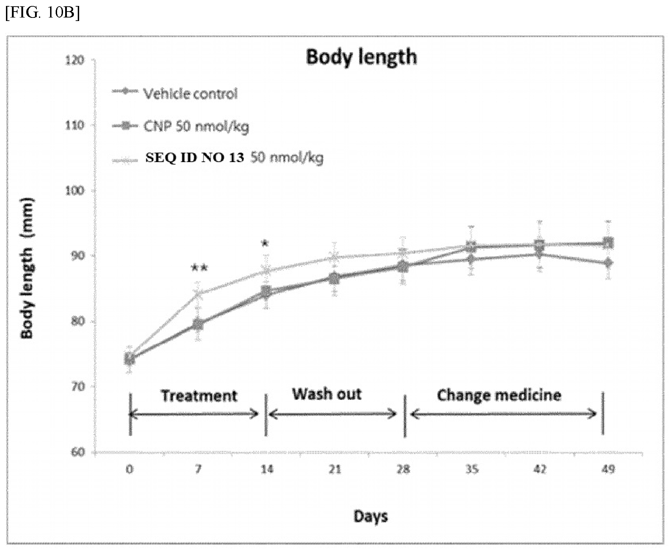

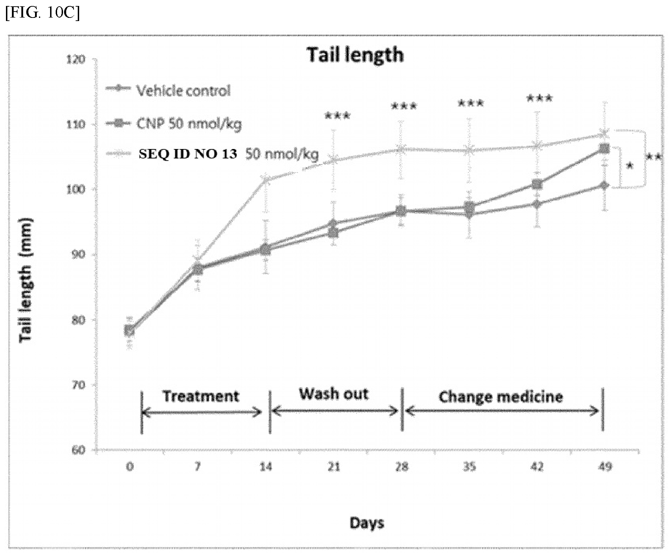

Specifically, the wild-type CNP (SEQ ID NO: 3) and the peptide of SEQ ID NO: 13 were each dissolved in an excipient (5% mannitol in saline), and then 3-week-old female ICR mice were subcutaneously injected daily with vehicle, CNP (50 nmol/kg), and the peptide of SEQ ID NO: 13 (50 nmol/kg) for 2 weeks, respectively. Thereafter, after a 2-week washout period, CNP (SEQ ID NO: 3) (50 nmol/kg) and the peptide of SEQ ID NO: 13 were crossover-administered to each group for 2 weeks, and the body weight, body length, and tail length of the mice were measured, and the measurement results were shown in Table 10 and FIGS. 10A to 10C.

**[Table 10]**

| First treatment | | Wash out | Second treatment (Change medicine) | |
|---|---|---|---|---|
| Group | 0~14 day | 14~28 day | Group | 28~49 day |
| Vehicle control | 0.9 ± 0.22 mm/day | 0.4 ± 0.14 mm/day | Vehicle control | 0.2 ± 0.09 mm/day |
| CNP 50 nmol/kg | 0.8 ± 0.26 mm/day | 0.4 ± 0.12 mm/day | SEQ ID NO: 13 50 nmol/kg | 0.4 ± 0.16*** mm/day |
| SEQ ID NO: 13 50 nmol/kg | 1.7 ± 0.24*** mm/day | 0.3 ± 0.12 mm/day | CNP 50 nmol/kg | 0.1 ± 0.08 mm/day |

| | | | | |
|---|---|---|---|---|
| - ***: p < 0.001 | | | | |

As shown in Table 10 and FIGS. 10A to 10C, the peptide of SEQ ID NO: 13, which was the CNP analogue according to an embodiment of the present disclosure, showed statistically significant growth differences in mouse body length and tail length for a period of 0 to 14 days compared to vehicle and wild-type CNP. In particular, it was found that the average daily growth was 1.7 mm from after treatment with the peptide of SEQ ID NO: 13 until day 14, whereas the control wild-type CNP (SEQ ID NO: 3) only grew by about 0.8 mm. In addition, even after cross-administration, the peptide of SEQ ID NO: 13 showed an average daily growth of 0.4 mm, while the control group, CNP, showed the growth of approximately 0.1 to 0.2 mm. It was confirmed that the CNP analogue of the present disclosure exhibited a high bone growth rate even upon cross-administration. This indicates that the CNP analogues of the present disclosure can be effectively used to treat bone-related diseases, including achondroplasia and osteoarthritis, or skeletal dysplasia.

### Example 12. Evaluation of mouse growth plate maintenance compared to positive control group (ASB20123) (3)

The growth plate maintenance and growth-promoting effects in normal mice were evaluated compared to a positive control group ASB20123 using the peptide of SEQ ID NO: 13, which had an excellent half-life among the CNP analogues prepared in Example 3.

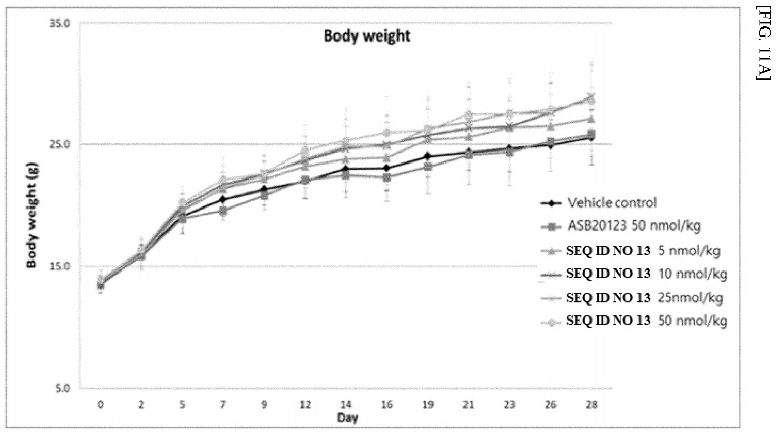

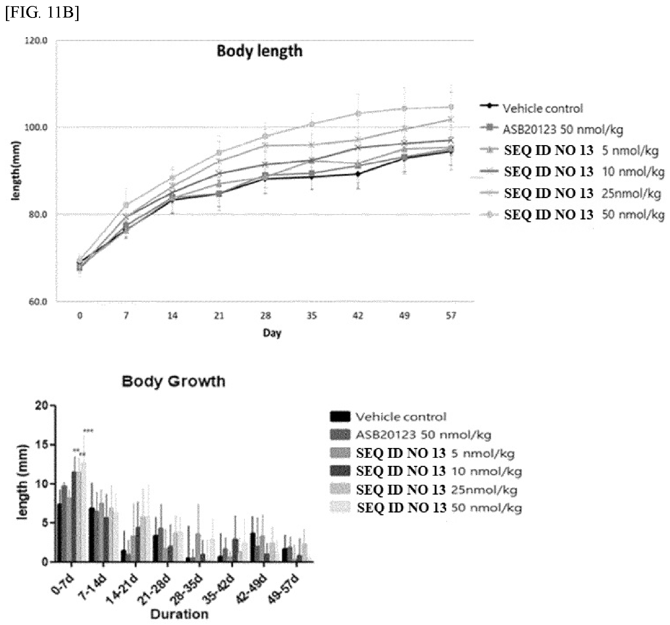

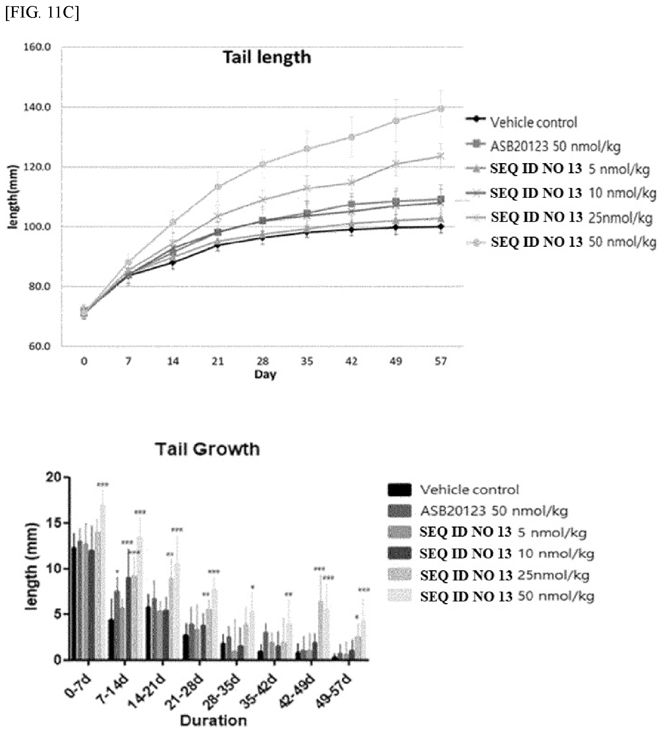

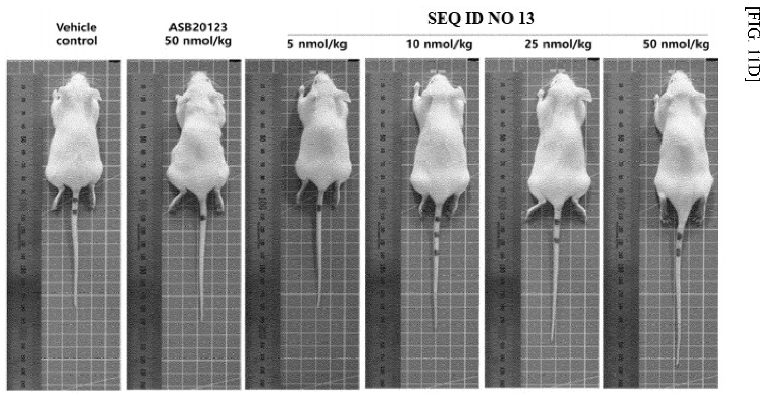

Specifically, the ASB20123 and the peptide of SEQ ID NO: 13 were each dissolved in an excipient (5% mannitol in saline), and then 3-week-old female ICR mice were subcutaneously injected daily with vehicle, ASB20123 (50 nmol/kg), and the peptide of SEQ ID NO: 13 (5, 10, 15, 20 nmol/kg) for 8 weeks, respectively. The body weight was measured twice a week and the body and tail lengths of the mouse were measured once a week, and the measurement results were shown in Tables 11 and 12, and FIGS. 11A to 11D. In Tables 11 and 12, each value represents the mean ± SD (mean ± standard deviation) of 7 to 8 mice, and *, **, and *** indicate p < 0.05, p < 0.01, and p < 0.001, respectively, when compared to a negative control group (vehicle control) using a Dunnett's test.

**[Table 11]**

| Group | Body length (mm) | | | | | | | |
|---|---|---|---|---|---|---|---|---|
| | 7 day | 14 day | 21 day | 28 day | 35 day | 42 day | 49 day | 57 day |
| Negative control (Vehicle control) | 76.4 ± 1.85 | 83.3 ± 3.06 | 84.8 ± 3.05 | 88.1 ± 2.97 | 89.3 ± 3.33 | 92.9 ± 3.18 | 92.9 ± 3.18 | 94.5 ± 3.21 |
| Positive control ASB20123 (50 nmol/kg) | 77.4 ± 1.13 | 83.9 ± 3.34 | 84.7 ± 3.82 | 89.0 ± 2.89 | 89.4 ± 2.76 | 91.1 ± 3.89 | 93.1 ± 3.89 | 95.0 ± 4.83 |
| SEQ ID NO: 13 (5 nmol/kg) | 76.3 ± 1.38 | 83.7 ± 1.11 | 87.0 ± 3.83 | 88.6 ± 4.00 | 92.3 ± 3.25 | 91.7 ± 3.64 | 95.0 ± 2.65 | 95.3 ± 1.80 |
| SEQ ID NO: 13 (10 nmol/kg) | 79.4 ± 2.33 | 85.0 ± 1.93 | 89.4 ± 3.16 | 91.4 ± 2.97 | 92.4 ± 3.50 | 95.3 ± 3.41 * | 96.3 ± 4.03 | 97.0 ± 3.46 |
| SEQ ID NO: 13 (15 nmol/kg) | 79.5 ± 2.00* | 86.4 ± 3.96* | 92.1 ± 4.49** | 95.8 ± 3.73*** | 95.9 ± 5.00*** | 97.1 ± 4.55** | 99.5 ± 5.01** | 101.8 ± 6.27** |
| SEQ ID NO: 13 | 82.1 ± | 88.4 ± | 94.1 ± | 97.9 ± | 100.8 ± | 103.1 ± | 104.3 ± | 104.6 ± |
| (20 nmol/kg) | 3.83*** | 2.50*** | 3.83*** | 3.14*** | 2.43 | 4.49*** | 4.89*** | 5.07*** |

**[Table 12]**

| Group | Tail length (mm) | | | | | | | |
|---|---|---|---|---|---|---|---|---|
| | 7 day | 14 day | 21 day | 28 day | 35 day | 42 day | 49 day | 57 day |
| Negative control (Vehicle control) | 83.6 ± 2.50 | 88.0 ± 2.14 | 93.8 ± 1.83 | 96.4 ± 2.26 | 98.1 ± 1.73 | 99.0 ± 2.00 | 99.8 ± 2.31 | 100.0 ± 2.07 |
| Positive control ASB20123 (50 nmol/kg) | 84.0 ± 1.63 | 91.4 ± 2.15 | 98.1 ± 3.44 | 102.0 ± 3.79* | 104.4 ± 3.99* | 107.4 ± 3.69** | 108.4 ± 4.35** | 109.1 ± 4.81*** |
| SEQ ID NO: 13 (5 nmol/kg) | 84.1 ± 1.34 | 89.9 ± 1.34 | 95.1 ± 1.07 | 97.4 ± 0.98 | 99.3 ± 1.50 | 101.1 ± 1.07 | 102.1 ± 1.68 | 1002.7 ± 1.60 |
| SEQ ID NO: 13 (10 nmol/kg) | 83.9 ± 3.56 | 92.9 ± 2.64** | 98.3 ± 3.54 | 102.0 ± 4.50* | 103.5 ± 4.17* | 105.0 ± 4.99* | 106.9 ± 4.82* | 107.9 ± 4.64** |
| SEQ ID NO: 13 (15 nmol/kg) | 85.4 ± 2.13 | 94.5 ± 3.50*** | 103.4 ± 3.85*** | 108.9 ± 3.18*** | 112.8 ± 4.27*** | 114.6 ± 2.45*** | 121.0 ± 4.11 *** | 123.5 ± 4.31*** |
| SEQ ID NO: 13 (20 nmol/kg) | 88.1 ± 2.95** | 101.5 ± 3.70*** | 113.3 ± 5.12*** | 120.9 ± 4.58*** | 126.0 ± 5.83*** | 129.9 ± 6.77*** | 135.4 ± 7.07*** | 139.5 ± 6.10*** |

As shown in Tables 11 and 12, and FIGS. 11A to 11D, in the case of the peptide of SEQ ID NO: 13, which was the CNP analogue according to an embodiment of the present disclosure, as the administration period increased, the growth of mouse body weight, body length, and tail length increased compared to the negative control group (vehicle) and the positive control group (ASB20123), and it was determined that the growth rate showed a concentration-dependent difference.

In particular, in the case of normal mice treated with the peptide of SEQ ID NO: 13 at a concentration of 20 nmol/kg for 8 weeks (57 days), the body length (mm) increased by approximately 10.1 mm compared to vehicle, and increased by approximately 9.6 mm compared to the positive control ASB20123, and the tail length (mm) increased by approximately 39.5 mm compared to vehicle and increased by approximately 30.4 mm compared to the positive control ASB20123.

From these results, it was found that the novel CNP analogue according to an embodiment of the present disclosure had significantly excellent effects of maintaining the growth plate and promoting bone growth.

The present disclosure has been described above with reference to preferred embodiments thereof. It is understood to those skilled in the art that the present disclosure may be implemented as a modified form without departing from an essential characteristic of the present disclosure. Therefore, the disclosed embodiments should be considered in an illustrative viewpoint rather than a restrictive viewpoint. The scope of the present disclosure is defined by the appended claims rather than by the foregoing description, and all differences within the scope of equivalents thereof should be construed as being included in the present disclosure.

## Claims

1. A C-type natriuretic peptide analogue having a structure of Formula 1 below:
[Formula 1] (P1)-PPKKGPPNG-(P2)
wherein in Formula 1,
P1 is a peptide consisting of 0 to 4 amino acids, and
P2 is a peptide consisting of an amino acid sequence of SEQ ID NO: 2 or 3, or a peptide comprising one or more amino acid substitutions in the amino acid sequence of SEQ ID NO: 2 or 3.

2. The C-type natriuretic peptide analogue of claim 1, wherein the P1 is represented by the following amino acid sequence:
P1 is (X1)-(X2)-(X3)-(X4),
X1 is Gly(G), Pro(P), Thr(T), Ala(A), Ser(S), or absent,
X2 is Asp(D), Gly(G), Lys(K), Ser(S), Asn(N), Glu(E), Gln(Q), or absent,
X3 is Asn(N), Lys(K), Asp(D), Ser(S), Gln(Q), or absent, and
X4 is Lys(K), Thr(T), Ser(S), Arg(R), Glu(E), Gln(Q), His(H), or absent.

3. The C-type natriuretic peptide analogue of claim 1, wherein the P2 is represented by the following Formula 2 (SEQ ID NO: 5):
[Formula 2] CFG-(X5)-K-(X6)-DRIGS-(X7)-SGLGC
wherein in Formula 2,
X5 and X7 are each selected from amino acids comprising cationic or hydrophobic side chains, and
X6 is selected from amino acids comprising cationic or hydrophobic side chains excluding Ile(I).

4. The C-type natriuretic peptide analogue of claim 3, wherein in Formula 2,
X5 is Leu(L) or His(H),
X6 is Leu(L), and
X7 is Met(M) or His(H).

5. The C-type natriuretic peptide analogue of claim 1, wherein a fatty acid or a derivative thereof is introduced in one or more repeating units into at least one amino acid among the amino acid sequence of the P1.

6. The C-type natriuretic peptide analogue of claim 5, wherein the fatty acid is a fatty acid having 3 to 21 carbon atoms, and is introduced directly or via a linker to the first to fourth amino acids from the N-terminus of the C-type natriuretic peptide analogue.

7. The C-type natriuretic peptide analogue of claim 6, wherein the fatty acid is selected from 8-amino-3,6-dioxaoctanoic acid, miniPEG, CH3(CH2)14COOH (palmitic acid), caprylic acid, capric acid, lauric acid, or stearic acid.

8. The C-type natriuretic peptide analogue of claim 1, wherein the C-type natriuretic peptide analogue comprise one or more amino acid sequences selected from the group consisting of SEQ ID NOs: 6 to 13.

9. The C-type natriuretic peptide analogue of any one of claims 1 to 8, wherein the C-type natriuretic peptide analogue improves one or more characteristics selected from the group consisting of *in vivo* half-life, bioavailability, and growth plate maintenance compared to a wild-type C-type natriuretic peptide.

10. A pharmaceutical composition for use in promoting growth comprising the C-type natriuretic peptide analogue according to any one of claims 1 to 8.

11. A food composition for use in promoting growth comprising the C-type natriuretic peptide analogue according to any one of claims 1 to 8.

12. A pharmaceutical composition for use in preventing or treating growth disorders comprising the C-type natriuretic peptide analogue according to any one of claims 1 to 8.

13. The pharmaceutical composition for use in preventing or treating growth disorders of claim 12, wherein the growth disorders comprise one or more selected from achondroplasia, dwarfism, bone-related diseases or bone dysplasia.

14. A method for promoting growth of a subject other than a human, comprising administering the C-type natriuretic peptide analogue according to any one of claims 1 to 8 to the subject other than the human.
